# EUROPEAN PATENT APPLICATION

(11) **EP 1 935 432 A1**
(43) Date of publication of application: **25.06.2008**
(21) Application number: 06810697.0
(22) Date of filing: 28.09.2006
(51) Int. Cl.: A61K 45/06, A61K 31/427, A61K 31/4985, A61P 3/10, A61P 43/00

(54) **MEDICAL AGENT CONTAINING INSULIN RESISTANCE IMPROVING AGENT**

(30) Priority: 29.09.2005 JP 2005283466
(71) Applicant: Daiichi Sankyo Company, Limited, Tokyo 103-8426 (JP)
(72) Inventor: KANDA, Shoichi, Shinagawa-ku Tokyo 140-8710 (JP); NAKASHIMA, Ryutaro, Shinagawa-ku Tokyo 140-8710 (JP)
(74) Representative: Gibson, Christian John Robert
(86) International application number: PCT/JP2006/319239
(87) International publication number: WO 2007/037296

(57) **Abstract**

To provide a method for treating diabetes which has an excellent blood glucose lowering action and causes minimal adverse drug reactions, there is provided a medicine comprising a DPP-IV inhibitor in combination with an insulin sensitizer.

## Description

### Technical Field

The present invention relates to a medicine (preferably, a therapeutic and/or prophylactic agent for diabetes) comprising a dipeptidyl peptidase IV inhibitor (DPP-IV inhibitor) in combination with an insulin sensitizer.

Furthermore, the present invention relates to the use of the above-mentioned compounds for the manufacture of the above-mentioned medicine and to a method for prophylactic or therapeutic treatment of the above-mentioned disease comprising administration of the above-mentioned medicine to a warm-blooded animal (preferably, a human).

### Background Art

Insulin sensitizers are administered to patients as therapeutic agents for diabetes to decrease blood glucose levels by improving impaired insulin action. Furthermore, reports have shown that these agents have prophylactic and therapeutic effects against not only diabetes, but also diseases attributable to insulin resistance such as hyperglycemia, impaired glucose tolerance, hypertension, hyperlipidemia, diabetic complications, gestational diabetes, and polycystic ovary syndrome and cardiovascular diseases such as atherosclerosis. Examples of currently marketed insulin sensitizers include pioglitazone, rosiglitazone, and so forth. It is thought that these agents exert the effect of improving impaired insulin action by activating peroxisome proliferator-activated receptor (PPAR) γ. 5-{4-[(6-Methoxy-1-methyl-1H-benzimidazol-2-yl)methoxy]benzyl}-1,3-thiazolidine-2,4-dione and pharmacologically acceptable salts thereof are also known to be insulin sensitizers having a PPAR γ activating action (refer to Patent Documents 1 and 2).

Meanwhile, DPP-IV inhibitors are envisaged as future therapeutic agents for diabetes because of their action of decreasing blood glucose levels and are disclosed in Patent Documents 3 to 6, for example.

Use of an insulin sensitizer in combination with a DPP-IV inhibitor is described in Patent Document 7, for example. However, the effects of combined use of the specific agents of the present invention are unknown.
[Patent Document 1]
   WO00/71540
[Patent Document 2]
   Japanese Patent Application No. 2005-14930
[Patent Document 3]
   WO05/3135
[Patent Document 4]
   WO03/4498
[Patent Document 5]
   WO00/34241
[Patent Document 6]
   WO01/68603
[Patent Document 7]
   WO01/97808

### Disclosure of the Invention

In recognition of the above-mentioned circumstances, the inventors of the present invention diligently conducted research for prophylactic and/or therapeutic agents for diabetes that cause minimal adverse drug reactions even through long-term drug treatment and that are effective in many diabetic patients. As a result, they found that the foregoing objects can be achieved by using a DPP-IV inhibitor in combination with an insulin sensitizer, and thus accomplished the present invention.

The present invention provides the following:
(1) A pharmaceutical composition comprising an insulin sensitizer in combination with a DPP-IV inhibitor;
(2) A pharmaceutical composition, characterized in that an insulin sensitizer is administered, and then a DPP-IV inhibitor is administered;
(3) A pharmaceutical composition, characterized in that adverse drug reactions of a DPP-IV inhibitor are reduced by using an insulin sensitizer in combination with the DPP-IV inhibitor;
(4) The pharmaceutical composition according to any one of the above (1) to (3), for prophylactic and therapeutic treatment of diabetes and having an enhanced blood glucose lowering action as compared with administration of either agent alone;
(5) The pharmaceutical composition according to any one of the above (1) to (4), wherein the DPP-IV inhibitor is a pyrazine or adamantyl DPP-IV inhibitor;
(6) The pharmaceutical composition according to any one of the above (1) to (4), wherein the DPP-IV inhibitor is a pyrazine DPP-IV inhibitor;
(7) The pharmaceutical composition according to any one of the above (1) to (4), wherein the DPP-IV inhibitor is MK-0431;
(8) The pharmaceutical composition according to any one of the above (1) to (7), wherein the insulin sensitizer is a thiazolidinedione insulin sensitizer;
(9) The pharmaceutical composition according to any one of the above (1) to (7), wherein the insulin sensitizer is a PPAR γ activator;
(10) The pharmaceutical composition according to any one of the above (1) to (7), wherein the insulin sensitizer is 5-{4-[(6-methoxy-1-methyl-1H-benzimidazol-2-yl)methoxy]benzyl}-1,3-thiazolidine-2,4-dione or a pharmacologically acceptable salt thereof;
(11) The pharmaceutical composition according to any one of the above (1) to (4), wherein the DPP-IV inhibitor is MK-0431, and the insulin sensitizer is 5-{4-[(6-methoxy-1-methyl-1H-benzimidazol-2-yl)methoxy]benzyl}-1,3-thiazolidine-2,4-dione or a pharmacologically acceptable salt thereof;
(12) The pharmaceutical composition according to any one of the above (1) to (11), for prophylactic or therapeutic treatment of a disease attributable to insulin resistance;
(13) The pharmaceutical composition according to any one of the above (1) to (11), for prophylactic or therapeutic treatment of diabetes;
(14) The pharmaceutical composition according to any one of the above (1) to (11), for improvement of glucose tolerance;
(15) The pharmaceutical composition according to any one of the above (1) to (14), for decreasing blood glucose levels;
(16) The pharmaceutical composition according to any one of the above (1) to (11), for prophylactic or therapeutic treatment of a cardiovascular disease;
(17) The pharmaceutical composition according to any one of the above (1) to (11), wherein the cardiovascular disease is atherosclerosis;
(18) The pharmaceutical composition according to any one of the above (1) to (17), which is a preparation for administering the respective agents at an interval;
(19) The pharmaceutical composition according to any one of the above (1) to (17), which is a preparation for administering the respective agents simultaneously;
(20) The pharmaceutical composition according to any one of the above (1) to (17), which is a preparation as a fixed combination drug;
(21) The pharmaceutical composition according to any one of the above (1) to (20), which is a preparation for oral administration;
(22) Use of an insulin sensitizer and a DPP-IV inhibitor for the manufacture of a medicine comprising an insulin sensitizer and a DPP-IV inhibitor as active ingredients;
(23) The use according to the above (22), wherein the DPP-IV inhibitor is MK-0431;
(24) The use according to the above (22) or (23), wherein the insulin sensitizer is a thiazolidinedione insulin sensitizer;
(25) The use according to the above (22) or (23), wherein the insulin sensitizer is 5- (4-[(6-methoxy-1-methyl-1H-benzimidazol-2-yl)methoxy]benzyl)-1,3-thiazolidine-2,4-dione or a pharmacologically acceptable salt thereof;
(26) The use according to the above (22), wherein the DPP-IV inhibitor is MK-0431, and the insulin sensitizer is 5-{4-[(6-methoxy-1-methyl-1H-benzimidazol-2-yl)methoxy]benzyl}-1,3-thiazolidine-2,4-dione or a pharmacologically acceptable salt thereof; and
(27) A method for treating diabetes, characterized in that an insulin sensitizer and a DPP-IV inhibitor are administered in combination.

In the present invention, the "insulin sensitizer" is a generic term for agents that improve insulin resistance and enhance insulin sensitivity, and examples thereof include pioglitazone (preferably, pioglitazone hydrochloride), rosiglitazone (preferably, rosiglitazone maleate), MCC-555, BMS-298585, AZ-242, LY-519818, R-483, MBX-102, AMG-131 (preferably, para-toluene sulfonates), 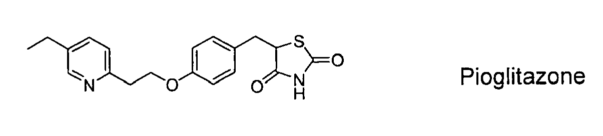 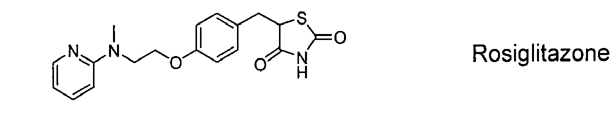 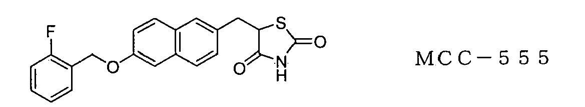 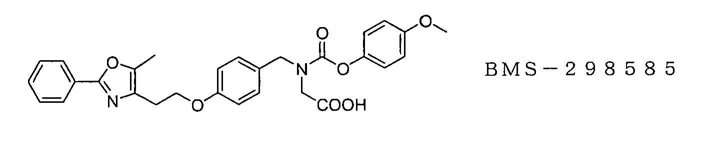 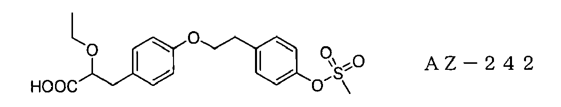 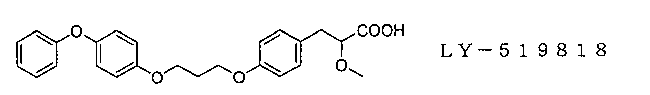 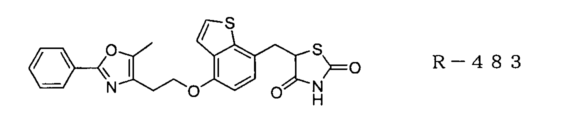 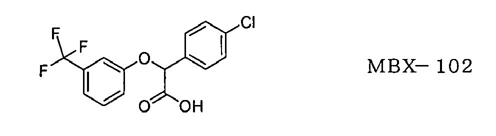 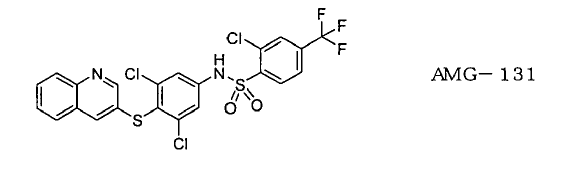 3-(2,4-dichlorobenzyl)-2-methyl-N-(pentylsulfonyl)-3H-benzimidazole-5-carboxamide (FK-614), 5-{4-[(6-methoxy-1-methyl-1H-benzimidazol-2-yl)methoxy]benzyl}-1,3-thiazolidine-2,4-dione (preferably, 5-{4-[(6-methoxy-1-methyl-1H-benzimidazol-2-yl)methoxy]benzyl}-1,3-thiazolidine-2,4-dione hydrochloride), and pharmacologically acceptable salts thereof. Preferred examples thereof include thiazolidinedione insulin sensitizers such as pioglitazone, rosiglitazone, 5-{4-[(6-methoxy-1-methyl-1H-benzimidazol-2-yl)methoxy]benzyl}-1,3-thiazolidine-2,4-dione, and pharmacologically acceptable salts thereof. The thiazolidinedione insulin sensitizers are known to improve insulin resistance by activating peroxisome proliferator-activated receptor (PPAR) γ and are also referred to as PPAR γ activators.

Pioglitazone is a compound described in U.S. Patent No. 4,687,777. Rosiglitazone is a compound described in U.S. Patent No. 5,002,953. MCC-555 is a compound described in U.S. Patent No. 5,594,016. BMS-298585 is a compound described in WO01/21602. AZ-242 is a compound described in WO99/62872. LY-519818 is a compound described in WO02/100813. 3-(2,4-Dichlorobenzyl)-2-methyl-N-(pentylsulfonyl)-3H-benzimidazole-5-carboxamide (FK-614) is a compound described in U.S. Patent No. 6,166,219. 5-{4-[(6-Methoxy-1-methyl-1H-benzimidazol-2-yl)methoxy]benzyl}-1,3-thiazolidine-2,4-dione and salts thereof are the compound represented by the following structure: 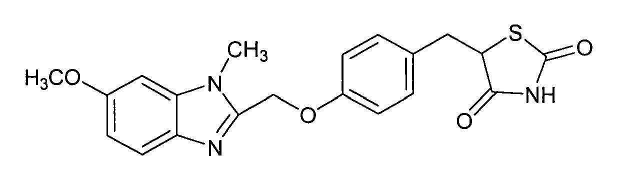 and pharmacologically acceptable salts thereof, and can be produced according to the methods described in Japanese Patent Laid-Open No. 9-295970, EP0745600, U.S. Patent No. 5,886,014, and WO00/71540.

In the present invention, the "dipeptidyl peptidase IV (DPP-IV) inhibitor" is not particularly limited so long as it is an agent that has actions such as inhibition of DPP-IV and suppression of degradation of GLP-1, and examples thereof include compounds represented by the structural formulas shown below, i.e., (2R)-4-oxo-4-[3-(trifluoromethyl)-5,6-dihydro[1,2,4]triazolo[4,3-α]pyrazin-7(8H)-yl]-1-(2,4,5-trifluorophenyl)butan-2-amine and pharmacologically acceptable salts thereof (MK-0431, etc.), which are pyrazine compounds having a pyrazine skeleton described in WO05/3135 and WO03/4498, (2S)-[[(3-hydroxyadamantan-1-yl)amino]acetyl]pyrrolidine-2-carbonitrile and pharmacologically acceptable salts thereof (LAF-237, etc.), which are adamantyl compounds having an adamantyl skeleton described in WO00/34241, (1S,3S,5S)-2-[(2S)-2-amino-2-(3-hydroxyadamantan-1-yl)acetyl]-2-azabicyclo[3.1.0]hexane-3-carbonitrile and pharmacologically acceptable salts thereof (BMS-477118, etc.), which are adamantyl compounds described in WO01/68603; and so forth. (2R)-4-oxo-4-[3-(trifluoromethyl)-5,6-dihydro[1,2,4]triazolo[4,3-α]pyrazin-7(8H)-yl]-1-(2,4,5-trifluorophenyl)butan-2-amine and pharmacologically acceptable salts thereof, which are pyrazine compounds, are preferred.

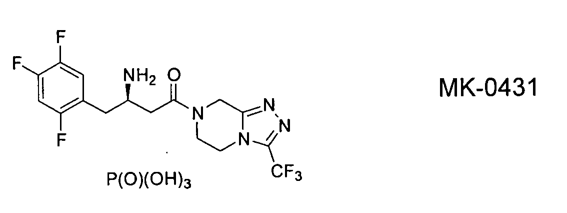

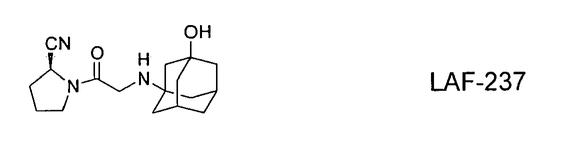

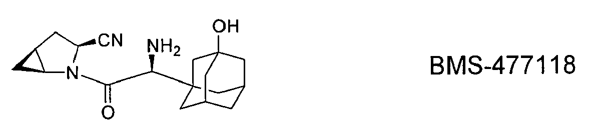

The DPP-IV inhibitor and insulin sensitizer can be administered in the form of a fixed combination drug. Furthermore, the individual agents can be simultaneously administered. Furthermore, the individual agents can be administered successively at a suitable interval. An interval that is acceptable to achieve an effect obtained by such agents can be confirmed by clinical or animal experiments.

The pharmaceutical composition of the present invention is administered in various dosage forms. The administration route thereof is not particularly limited and is determined depending on the dosage form, patient's age, sex, and other conditions, severity of the disease, and the like. For example, the pharmaceutical composition of the present invention is orally administered in the form of tablets, pills, powders, granules, syrups, solutions, suspensions, emulsions, granules, or capsules.

These various preparations can be prepared according to conventional methods using known auxiliaries usually used in the known field of pharmaceutical preparations such as excipients, binders, disintegrants, lubricants, solubilizing agents, flavoring agents, coating agents, in addition to the active ingredients.

For preparing in the form of tablets, a broad range of known carriers can be used, and examples thereof include excipients such as lactose, sucrose, sodium chloride, glucose, urea, starch, calcium carbonate, kaolin, crystalline cellulose, and silicic acid, binders such as water, ethanol, propanol, simple syrup, glucose solution, starch solution, gelatin solution, carboxymethylcellulose, shellac, methylcellulose, potassium phosphate, and polyvinylpyrrolidone, disintegrants such as dry starch, sodium alginate, powdered agar, laminaran powder, sodium hydrogen carbonate, calcium carbonate, polyoxyethylene sorbitan fatty acid esters, lauryl sodium sulfate, monoglyceride stearate, starch, and lactose, disintegration suppressing agents such as sucrose, stearin, cocoa butter, and hydrogenated oil, absorption promoters such as quaternary ammonium base and lauryl sodium sulfate, moisturizing agents such as glycerine and starch, adsorbents such as starch, lactose, kaolin, bentonite, and colloidal silicic acid, lubricants such as purified talc, stearates, boric acid powder, and polyethylene glycol, and so forth. Furthermore, tablets can be prepared as tablets coated with a usual tablet coating, for example, sugar-coated tablets, gelatin-coated tablets, enteric-coated tablets, film-coated tablets, double-layered tablets, or multi-layered tablets, as required.

For preparing in the form of pills, a broad range of carriers known in this field can be used, and examples thereof include excipients such as glucose, lactose, starch, cocoa butter, hydrogenated vegetable oil, kaolin, and talc, binders such as powdered gum arabic, powdered tragacanth, gelatin, and ethanol, disintegrants such as laminaran, agar, and so forth.

Furthermore, if necessary, coloring materials, preservatives, flavors, flavoring agents, sweeteners, and the like, or other pharmaceutical products may be added.

The dose of each therapeutic agent for diabetes used in the present invention and the dosing ratio greatly vary depending on various conditions such as the activity of each substance, patient's symptoms, age, and body weight.

The amount of insulin sensitizer contained in the above-mentioned pharmaceutical preparation is not particularly limited and is suitably selected from a broad range. An appropriate content is usually 1 to 70% by weight, preferably 1 to 30 % by weight, of the total composition.

The dose varies depending on the symptoms, age, body weight, dosage form, and the like, and for a usual daily dose for adults the lower limit and the upper limit are 0.0001 mg/kg (preferably 0.001 mg/kg, more preferably 0.01 mg/kg) and 30 mg/kg (preferably 3 mg/kg, more preferably 0.3 mg/kg), respectively, which can be administered as one or two doses.

The amount of DPP-IV inhibitor contained in the above-mentioned pharmaceutical preparation is not particularly limited and is suitably selected from a broad range. An appropriate content is usually 1 to 70% by weight, preferably 1 to 30 % by weight, of the total composition.

The dose varies depending on the symptoms, age, body weight, dosage form, and the like, and for a usual daily dose for adults the lower limit and the upper limit are 0.0001 mg/kg (preferably 0.001 mg/kg, more preferably 0.01 mg/kg) and 30 mg/kg (preferably 3 mg/kg, more preferably 0.3 mg/kg), respectively, which can be administered as one to three doses.

In the present invention, the above-mentioned doses of the DPP-IV inhibitor and the insulin sensitizer are administered once daily, or divided into several doses and administered simultaneously or separately at different times.

According to the present invention, an excellent blood glucose lowering action is exhibited against high blood glucose levels in diabetes by using a DPP-IV inhibitor in combination with an insulin sensitizer, and diabetes can thereby be prevented or treated effectively. Furthermore, this medicine is also effective for prophylactic and therapeutic treatment of diabetic complications attributable to high blood glucose levels, diseases attributable to insulin resistance such as hyperglycemia, impaired glucose tolerance, hypertension, hyperlipidemia, diabetic complications, gestational diabetes, and polycystic ovary syndrome, and cardiovascular diseases such as atherosclerosis. Furthermore, rapid improvement of high blood glucose levels and a stable blood glucose lowering action through long-term administration are expected by suitably selecting the type of each agent, administration method, doses, and the like depending on the symptoms, and this medicine can be a prophylactic and therapeutic agent for the above-mentioned diseases with very few adverse drug reactions.

### Brief Description of the Drawing

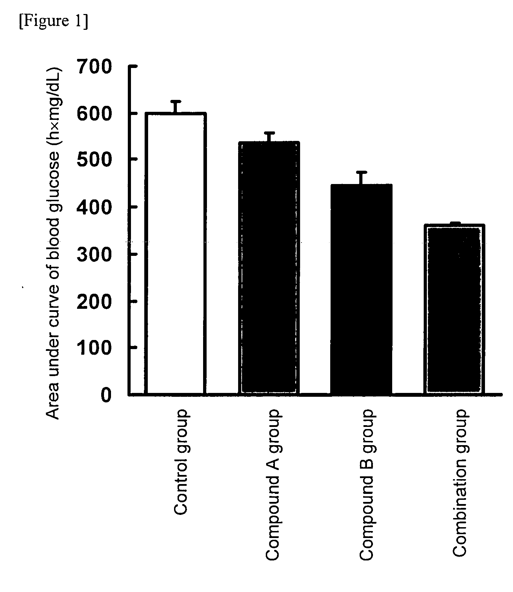

Figure 1 shows a glucose tolerance improving effect by combined use of 5-{4-[(6-methoxy-1-methyl-1H-benzimidazol-2-yl)methoxy]benzyl}-1,3-thiazolidine-2,4-dione hydrochloride (Compound A) and MK-0431(Compound B) (Test Example 1).

### Best Mode for Carrying Out the Invention

The present invention will be explained more specifically with reference to the following examples. However, the scope of the present invention is not limited to these examples.

### Examples

5-{4-[(6-Methoxy-1-methyl-1H-benzimidazol-2-yl)methoxy]benzyl}-1,3-thiazolidine-2,4-dione hydrochloride (Compound A), an insulin sensitizer, can be produced according to the methods described in Japanese Patent Laid-Open No. 9-295970, EP0745600, U.S. Patent No. 5,886,014, and WO00/71540. MK-0431 (Compound B), a DPP-IV inhibitor, can be produced according to the methods described in WO05/3135 and WO03/4498.

### Test Example 1

### Glucose tolerance improving effect by combined use of 5-{4-[(6-methoxy-1-methyl-1H-benzimidazol-2-yl)methoxy]benzyl}-1,3-thiazolidine-2,4-dione hydrochloride (Compound A) and MK-0431 (Compound B)

12-week-old male obese Zucker fatty rats (Charles River Laboratories Japan, Inc.) with severe insulin resistance and impaired glucose tolerance were assigned to each of four groups, i.e., a control group, a Compound A group, a Compound B group, and a combination group (5 animals/group). Feed (FR-2, Funabashi Farms Co., Ltd.) was available ad libitum. The Compound A group and the combination group were given repeated oral doses of 0.02 mg/kg of Compound A, an insulin sensitizer, for one week, the control group and the Compound B group were given a vehicle alone for the same period, and then all the groups were fasted overnight. After fasting, a 50% glucose solution (Otsuka Pharmaceutical Factory, Inc.) was orally given at a dose of 2 g/kg to perform a glucose tolerance test. The Compound B group and the combination group were given 5 mg/kg of Compound B, a DPP-IV inhibitor, one hour before the glucose load, and blood samples were collected from the caudal vein of all the individual animals immediately before and 0.5, 1, 1.5, and 2 hours after the glucose load to measure blood glucose levels using a fully automated glucose analyzer (Glucoroder-GXT, A&T). The area under curve of blood glucose was calculated for each individual animal with values obtained by substracting the blood glucose level immediately before administration from the blood glucose levels measured at each time point. The mean area under curve of blood glucose and the standard error for each group were obtained from these values as shown in Figure 1. A greater decrease in the area under curve of blood glucose means a higher blood glucose lowering action.

As shown in Figure 1, it can be confirmed that combined use of Compound A, an insulin sensitizer, and Compound B, a DPP-IV inhibitor, decreases the area under curve of blood glucose without attenuating each other's effect (by two-way analysis of variance). This result suggests that the blood glucose lowering action was enhanced by the combined use of Compound A and Compound B.

### Test Example 2

### Glucose tolerance improving effect by combined use of 5-{4-[(6-methoxy-1-methyl-1H-benzimidazol-2-yl)methoxy]benzyl}-1,3-thiazolidine-2,4-dione hydrochloride (Compound A) and MK-0431 (Compound B)

12-week-old male obese Zucker fatty rats (Charles River Laboratories Japan, Inc.) with severe insulin resistance and impaired glucose tolerance are given repeated oral doses of 0.02 mg/kg of Compound A, an insulin sensitizer, for one week (feed: FR-2, Funabashi Farms Co., Ltd.), fasted overnight, and orally given a 50% glucose solution (Otsuka Pharmaceutical Factory, Inc.) at a dose of 2 g/kg to perform a glucose tolerance test. The effect of combined use of Compound A and Compound B is examined by giving 2 mg/kg of compound B, a DPP-IV inhibitor, one hour before the glucose load. Drug efficacy is evaluated by blood glucose levels up to two hours after the glucose load. The group organization is as follows.

**Table 1**

| | 1-week repeated administration | Administration before glucose load | No. of animals |
|---|---|---|---|
| (i) Control group | Vehicle | Vehicle | 5 |
| (ii) Compound A group | Compound A | Vehicle | 5 |
| (iii) Compound B group | Vehicle | Compound B | 5 |
| (iv) Combination group | Compound A | Compound B | 5 |

It can be confirmed that, the area under curve of blood glucose decreases in the combination group given Compound A, an insulin sensitizer, and Compound B, a DPP-IV inhibitor, without attenuating each other's effect (by two-way analysis of variance).

The area under curve of blood glucose after administration of glucose is an indicator of glucose tolerance, and an increase in the area under curve indicates impairment of glucose tolerance. Impairment of glucose tolerance is one of the diagnostic criteria of diabetes, and improvement of this condition leads to treatment of diabetes. Therefore, the medicine of the present invention is useful for prophylactic and therapeutic treatment of diabetes because it improves glucose tolerance more effectively than treatment with one agent alone. Furthermore, since an adequate effect can be obtained at lower doses of the medicine of the present invention as compared with treatment with each agent alone, adverse drug reactions that appear to be caused by DPP-IV inhibitors (e.g., anorexia, nausea, liver dysfunction, immunodeficiency, etc.) can be reduced in treatment of diabetes or the like.

### Preparation examples

### (1) Capsule

| | |
|---|---|
| Compound A | 1 mg |
| Compound B | 25 mg |
| Lactose | 75 mg |
| Corn starch | 57 mg |
| Magnesium stearate | 2 mg |
| Total | 160 mg |

All the powder components shown above are well mixed, and sifted through a sieve. 160 mg of the obtained powder is weighed and filled into a gelatin capsule to prepare a capsule.

### (2) Tablet

| | |
|---|---|
| Compound A | 1 mg |
| Compound B | 25 mg |
| Lactose | 35 mg |
| Corn starch | 33 mg |
| Microcrystalline cellulose | 20 mg |
| Magnesium stearate | 1 mg |
| Total | 115 mg |

All the powder components shown above are well mixed, and compressed and molded into tablets each having a weight of 115 mg. If necessary, these tablets may be coated with sugar or a film.

## Claims

1. A pharmaceutical composition comprising an insulin sensitizer in combination with a DPP-IV inhibitor.

2. A pharmaceutical composition, **characterized in that** an insulin sensitizer is administered, and then a DPP-IV inhibitor is administered.

3. A pharmaceutical composition, **characterized in that** adverse drug reactions of a DPP-IV inhibitor are reduced by using an insulin sensitizer in combination with the DPP-IV inhibitor.

4. The pharmaceutical composition according to any one of claims 1 to 3, for prophylactic and therapeutic treatment of diabetes and having an enhanced blood glucose lowering action as compared with administration of either agent alone.

5. The pharmaceutical composition according to any one of claims 1 to 4, wherein the DPP-IV inhibitor is a pyrazine or adamantyl DPP-IV inhibitor.

6. The pharmaceutical composition according to any one of claims 1 to 4, wherein the DPP-IV inhibitor is a pyrazine DPP-IV inhibitor.

7. The pharmaceutical composition according to any one of claims 1 to 4, wherein the DPP-IV inhibitor is MK-0431.

8. The pharmaceutical composition according to any one of claims 1 to 7, wherein the insulin sensitizer is a thiazolidinedione insulin sensitizer.

9. The pharmaceutical composition according to any one of claims 1 to 7, wherein the insulin sensitizer is a PPAR γ activator.

10. The pharmaceutical composition according to any one of claims 1 to 7, wherein the insulin sensitizer is 5-{4-[(6-methoxy-1-methyl-1H-benzimidazol-2-yl)methoxy]benzyl}-1,3-thiazolidine-2,4-dione or a pharmacologically acceptable salt thereof.

11. The pharmaceutical composition according to any one of claims 1 to 4, wherein the DPP-IV inhibitor is MK-0431, and the insulin sensitizer is 5-{4-[(6-methoxy-1-methyl-1H-benzimidazol-2-yl)methoxy]benzyl}-1,3-thiazolidine-2,4-dione or a pharmacologically acceptable salt thereof.

12. The pharmaceutical composition according to any one of claims 1 to 11, for prophylactic or therapeutic treatment of a disease attributable to insulin resistance.

13. The pharmaceutical composition according to any one of claims 1 to 11, for prophylactic or therapeutic treatment of diabetes.

14. The pharmaceutical composition according to any one of claims 1 to 10, for improvement of glucose tolerance.

15. The pharmaceutical composition according to any one of claims 1 to 14, for decreasing blood glucose levels.

16. The pharmaceutical composition according to any one of claims 1 to 11, for prophylactic or therapeutic treatment of a cardiovascular disease.

17. The pharmaceutical composition according to any one of claims 1 to 11, wherein the cardiovascular disease is atherosclerosis.

18. The pharmaceutical composition according to any one of claims 1 to 17, which is a preparation for administering the respective agents at an interval.

19. The pharmaceutical composition according to any one of claims 1 to 17, which is a preparation for administering the respective agents simultaneously.

20. The pharmaceutical composition according to any one of claims 1 to 17, which is a preparation as a fixed combination drug.

21. The pharmaceutical composition according to any one of claims 1 to 20, which is a preparation for oral administration.

22. Use of an insulin sensitizer and a DPP-IV inhibitor for the manufacture of a medicine comprising an insulin sensitizer and a DPP-IV inhibitor as active ingredients.

23. The use according to claim 22, wherein the DPP-IV inhibitor is MK-0431.

24. The use according to claim 22 or 23, wherein the insulin sensitizer is a thiazolidinedione insulin sensitizer.

25. The use according to claim 22 or 23, wherein the insulin sensitizer is 5-{4-[(6-methoxy-1-methyl-1H-benzimidazol-2-yl)methoxy]benzyl}-1,3-thiazolidine-2,4-dione or a pharmacologically acceptable salt thereof.

26. The use according to claim 22, wherein the DPP-IV inhibitor is MK-0431, and the insulin sensitizer is 5-{4-[(6-methoxy-1-methyl-1H-benzimidazol-2-yl)methoxy]benzyl}-1,3-thiazolidine-2,4-dione or a pharmacologically acceptable salt thereof.

27. A method for treating diabetes, **characterized in that** an insulin sensitizer and a DPP-IV inhibitor are administered in combination.
